# EUROPÄISCHE PATENTANMELDUNG

(11) **EP 4 249 460 A1**
(43) Veröffentlichungstag der Anmeldung: **27.09.2023**
(21) Anmeldenummer: 22164368.7
(22) Anmeldetag: 25.03.2022
(51) Int. Cl.: C07C 29/70, C07C 31/30

(54) **VERFAHREN ZUR HERSTELLUNG VON MAGNESIUMETHOXID**

(71) Anmelder: LANXESS Organometallics GmbH, 59192 Bergkamen (DE)
(72) Erfinder: Haneke, Lukas, 48653 Coesfeld (DE); Holtrichter- Rößmann, Thorsten, 59227 Ahlen (DE); Liedtke, Claus Günter, 59368 Werne (DE)
(74) Vertreter: Deblon, Jörg-Stephan

(57) **Zusammenfassung**

Die vorliegende Erfindung betrifft ein Verfahren zur Herstellung von Magnesiumethoxid aus der Reaktion von Magnesium und Ethanol in Gegenwart eines Aluminiumalkyls als Aktivator in einem inerten organischen Lösungsmittel.

## Beschreibung

Die vorliegende Erfindung betrifft ein Verfahren zur Herstellung von Magnesiumethoxid aus der Reaktion von Magnesium und Ethanol in Gegenwart eines Aluminiumalkyls als Aktivator in einem geeigneten organischen Lösungsmittel.

Magnesiumethoxid (Magnesiumdiethoxid; Magnesiumbisethanolat; CAS#: 2414-98-4; Mg(OEt)₂; Mg(OCH₂CH₃)₂) ist eine wichtige Ausgangsverbindung für die chemische Industrie und hat große Bedeutung für Werkstoffe und Beschichtungen, u.a. in der Elektronik. Außerdem findet es Anwendung als Ausgangsstoff (Precursor) für die Herstellung von Magnesiumoxid (MgO) mittels Sol-Gel-Hydrolyse für Hochtemperatursupraleiter,zur Herstellung weiterer Magnesiumalkoxide sowie für Keramiken und Buchkonservierungsmittel.

Magnesiumethoxid ist wird weiterhin in industriellem Maßstab auch für die Herstellung von sehr reinem Magnesiumchlorid (MgCl₂) verwendet, wo es als aktiver Träger für TiCl₄/AlR₃ Ziegler-Natta-Polymerisationskatalysatoren in der Polyolefin-Synthese, bspw. von Polyethylen (PE) oder Polypropylen (PP) zum Einsatz kommt.

Magnesiumethoxid ist luft- und feuchtigkeitsempfindlich. Mit Wasser hydrolysiert es zu Magnesiumhydroxid (Mg(OH)₂) und Ethanol; mit Sauerstoff wird es zu Acetaldehyd und Mg(OH)₂ oxidiert. Magnesiumethoxid ist unlöslich in unpolaren Kohlenwasserstoffen wie Toluol und Hexan sowie in chlorierten Kohlenwasserstoffen. Auch in Ethanol ist Magnesiumethoxid zu weniger als 0,2 Gew.-% bei 20°C löslich.

Magnesiumethoxid wird üblicherweise durch Umsetzung von elementarem Magnesium mit Ethanol (EtOH) unter Abspaltung von Wasserstoff gemäß der Reaktion ((I) synthetisiert:

Mg + 2 EtOH → Mg(OEt)₂ + H₂ (I)

Aus dem Stand der Technik sind zahlreiche Varianten davon und weitere Verfahren zur Herstellung von Magnesiumethoxid bekannt.

DE-A-2261386 offenbart Verfahren zur Herstellung von Magnesiumethoxid durch Umsetzung von Magnesium mit Ethanol bei Temperaturen von 100 bis 200°C in Gegenwart von Toluol. Das hergestellte Magnesiumethoxid wird im Anschluss filtriert wobei das Magnesiumethoxid in Form von Brocken vorliegt. Das Verhältnis Toluol zu Ethanol beträgt 1:5 bis 1:20.

DE-A-1 806 549 offenbart Verfahren zur Herstellung von Magnesiumethoxid durch Umsetzen von Magnesiumspänen mit Ethanol in Gegenwart von Orthoameisensäure und p-Toluolsulfonsäure. Der Magnesiumgehalt des hergestellten Magnesiumethoxids beträgt 21,4 Gew.-% und der Mg(OH)₂-Gehalt 0,16 Gew.-%.

DE-A-19849353 offenbart Verfahren zur Herstellung von Magnesiumethoxid mit hohem Grobkornanteil durch Umsetzung von metallischem, gegebenenfalls aktiviertem Magnesium mit flüssigem Ethanol unter Druck bei einer Temperatur oberhalb 78°C ohne weiteres Lösungsmittel und anschließender Gewinnung des Magnesiumethoxids.

US-A-4,876,230 offenbart Verfahren zur Herstellung von Magnesiumethoxid durch Umsetzung von Magnesiumpulver mit Ethanol in Gegenwart von Tetraethoxysilan und Eisenchlorid-Lösung.

WO-A-2015177731 offenbart Verfahren zur Herstellung von Magnesiumethoxid umfassend die Umsetzung von Magnesium mit Ethanol in Gegenwart eines Aktivators (I₂, TiCl₄, MgCl₂) für eine Zeit von 30 Minuten bis 3 Stunden, gefolgt von einer Nachreaktion bei 65 bis 80°C für 30 Minuten bis 10 Stunden und anschließender Trocknung bei 60 bis 120°C. Die Reaktion wird in Abwesenheit eines inerten organischen Lösungsmittels durchgeführt.

DE-A-102006041315 offenbart aktiviertes Magnesium, das durch Zugabe einer aktivierenden Verbindung wie beispielsweise Triethylaluminium (TEA) in einem nichtkoordinierenden Lösungsmittel zum Erdalkalimetall erhältlich ist und dessen Verwendung zur Herstellung von Erdalkalialkoxiden. Weiterhin wird ein Verfahren offenbart, bei dem im ersten Schritt Magnesiumfeinspäne in Toluol unter Rühren bei 50°C mit einer 25%ige Triethylaluminiumlösung aktiviert werden. Im zweiten Schritt wird zur Umsetzung des aktivierten Magnesiums Ethanol im Überschuss über einem Zeitraum von 1,5 Stunden zugegeben. Das Verhältnis von Ethanol zu Toluol beträgt von 1:2,1 bis 1:3. Nach einer Nachreaktionszeit von 100 Minuten ist die Reaktion abgeschlossen und das Magnesiumethoxid wird gefiltert.

Für die Herstellung von Ziegler-Natta-Katalysatoren ist aus EP-A-1031580 bekannt Magnesiumethoxid in Form sphärischer Partikel durch Reaktion mit Titanchlorid oder einer anderen Titan-Halogenbindungen aufweisenden Verbindung (z.B. Cp₂TiCl₂) gemäß der Reaktion (II) in eine aktive Form zu überführen.

Mg(OEt)₂ + Cp₂TiCl₂ → Mg(OEt)₂₋ₓClₓ + Cp₂TiCl₂₋ₓ(OEt)ₓ x = 0 bis 2 (II)

Insbesondere für vorgenannte Anwendungen werden hohe Anforderungen an die Reinheit und Beschaffenheit von Magnesiumethoxid gestellt, bspw. eine Reinheit von ≥ 98,6 Gew.-%, Partikeldurchmesser von 70-90 % von 500 bis 800 µm, ein Gehalt an freiem Ethanol von ≤ 0,6 Gew.-% und ein Gehalt an elementarem Magnesium von ≤05 Gew.-%.

Der Anteil an ungelösten Oxidations- und Nebenprodukten wie Mg(OH)₂ oder MgCO₃ sollte möglichst gering sein, bevorzugt ≤ 1,0 Gew.-% bezogen auf das Gesamtgewicht des Magnesiumethoxid, da diese den Nachteil haben, dass sie durch Filtration oder weiterelsolationsmethoden wie Kristallisation äußerst schwierig abzutrennen sind.

Das Magnesiumethoxid soll aufgrund seiner Verwendung in Ziegler-Natta-Katalysatoren keine weiteren Verunreinigungen, wie beispielsweise von Aktivatorenwie insbesondere Iod aufweisen, die sich nachteilig auf die Polymerisationsinitiation oder Anwendungseigenschaften auswirken.

Der Stand der Technik zeigt, dass es nicht ohne weiteres möglich war Mg(OEt)₂ herzustellen, welches den hohen Anforderungen zur Herstellung von MgCl₂ gerecht wird, insbesondere die zuvor genannte Spezifikation erfüllt. Die Herstellungsverfahren des Standes der Technik durch viele Probleme gekennzeichnet, insbesondere hinsichtlich der Partikelgröße. Eine zu große Partikelgröße des hergestellten Magnesiumethoxids bewirkt, dass es zu einer sogenannten Verbackung, das heißt einem "Absetzen" des Magnesiumethoxids im Reaktionsgefäßes kommt, welches einerseits den weiteren Reaktionsprozess hindert und andererseits einen hohen apparativen Aufwand zur Isolierung aus dem Reaktionsgefäß erfordert.

Eine zu kleine Partikelgröße des hergestellten Magnesiumethoxids bewirkt hingegen, dass bereits im Herstellungsprozess der Filter für die Abtrennung des Magnesiumethoxids verstopft wird und aufwendig gereinigt werden muss.

Die Partikelgröße des hergestellten Magnesiumethoxids ist nicht nur abhängig von der eingesetzten Größe an elementaren Magnesium und kann somit nicht ohne Weiteres gesteuert werden.

Es bestand somit weiterhin ein seit langem bestehendes Bedürfnis nach einem einfachen Herstellungsverfahren, welches die vorgenannten Anforderungen erfüllt.

Die überraschenderweise gefundene Lösung der Aufgabe und Gegenstand der vorliegenden Erfindung ist nun ein Verfahren zur Herstellung von Magnesiumethoxid umfassend die Schritte
(1) Inkontaktbringen von elementarem Magnesium mit zumindest einem Aluminiumalkyl in Gegenwart von inertem organischen Lösungsmittel und
(2) Zugabe von Ethanol bei einer Temperatur von 40 bis 70 °C,
wobei das molare Verhältnis von Ethanol zu inertem organischen Lösungsmittel von 1:0,7 bis 1:2, bevorzugt von 1:0,8 bis 1:1,8 und besonders bevorzugt von 1:1 bis 1:1,5 beträgt.

Es sei an dieser Stelle angemerkt, dass der Rahmen der Erfindung alle beliebigen und möglichen Kombinationen der oben stehenden und im Folgenden aufgeführten, allgemeinen oder in Vorzugsbereichen genannten Komponenten, Wertebereiche bzw. Verfahrensparameter umfasst.

Das erfindungsgemäße Verfahren zur Herstellung des aktivierten Magnesiums wird allgemein wie nachfolgend beschrieben durchgeführt, ohne die Erfindung darauf einzuschränken.

In Schritt (1) des erfindungsgemäßen Verfahrens erfolgt das Inkontaktbringen von elementarem Magnesium mit zumindest einem Aluminiumalkyl in Gegenwart eines inerten organischen Lösungsmittels.

Das beim erfindungsgemäßen Verfahren eingesetzte elementare Magnesium kann beispielsweise in Form von Magnesiumpulver, Magnesiumgranulat oder Magnesiumspänen vorliegen, bevorzugt in Form von Magnesiumspänen.

Magnesiumpulver weist bevorzugt eine durchschnittliche Partikelgröße bestimmt mittels Siebanalyse nach ISO 4497 von 20 bis 5.000 µm auf.

Geeignete Magnesiumspäne sind kommerziell erhältlich und weisen bevorzugt eine Dicke von 0,1 bis 0,5 mm, eine Breite von 0,5 bis 1,5 mm und eine Länge von 2 bis 4 mm auf.

Magnesiumpulver besitzt typischerweise eine größere Oberfläche im Vergleich zu Magnesiumspänen und führt daher zu einer schnelleren Reaktion als Magnesiumspäne.

Magnesiumspäne führen nach dem erfindungsgemäßen Verfahren zu Magnesiumethoxid-Partikeln mit ca. 2 mm Durchmesser.

Das für das erfindungsgemäße Verfahren eingesetzte inerte organische Lösungsmittel ist definitionsgemäß eines, das unter den gewählten Reaktionsbedingungen nicht oder nicht merklich mit Aluminiumalkylen, Magnesium oder Ethanol reagiert und umfasst aromatische oder aliphatische Kohlenwasserstoffe oder Mischungen davon. Bevorzugte inerte, organische Lösungsmittel, sind Toluol, n-Octan, Heptane; Hexane, Cyclohexan, Pentane, Paraffinöle der beliebige Mischungen davon, besonders bevorzugt ist Toluol.

Im erfindungsgemäßen Verfahren beträgt die Menge an inertem organischen Lösungsmittel beispielsweise 1 bis 10 Mol-Äquivalente, bevorzugt 2 bis 7 Mol-Äquivalente und besonders bevorzugt 2 bis 4 Mol-Äquivalente bezogen auf die Menge an eingesetztem Magnesium.

Die für das erfindungsgemäße Verfahren eingesetzten Aluminiumalkyle können beispielsweise Trimethylaluminium (TMA), Triethylaluminium (TEA), Tributylaluminium (TBA), Triisobutylaluminium (TiBA), Ethylaluminiumdichlorid (EADC) oder Diethylaluminiumchlorid (DEAC) sein. Bevorzugt wird Triethylaluminium (TEA) eingesetzt.

Im erfindungsgemäßen Verfahren beträgt die Menge an Aluminiumalkyl beispielsweise 0,001 bis 0,1 Äquivalente, bevorzugt 0,6 bis 0,8 Mol-Äquivalente bezogen auf die Menge Magnesium.

Ohne wissenschaftliche Festlegung wird vermutet, dass Aluminiumalkyle mit dem im inerten organischen Lösungsmittel enthaltenen Wasserspuren reagieren, die ansonsten mit Magnesium eine passivierende Mg(OH)₂/MgO Schicht bilden würden. Es wird weiterhin vermutet, dass Aluminiumalkyle auch mit der passivierenden Oxidschicht des Magnesiums selbst reagieren und insgesamt so als Aktivator fungieren.

In Schritt (2) des erfindungsgemäßen Verfahrens erfolgt die Zugabe von Ethanol zur erhaltenen Zusammensetzung aus Schritt (1) bei einer Temperatur von 40 bis 70 °C. Hierbei wird das Magnesium mit Ethanol umgesetzt.

In einer bevorzugten Ausführungsform weist das eingesetzte Ethanol einen Wassergehalt von weniger als 0,05 Gew.-%, besonders bevorzugt weniger als 0,01 Gew.-% und ganz besonders bevorzugt weniger als 0,01 Gew.-% bezogen auf das Gesamtgewicht des Ethanols auf.

Im erfindungsgemäßen Verfahren beträgt das molare Verhältnis von Magnesium zu Ethanol beispielsweise1:2,1 bis 1:4, bevorzugt 1:2,5 bis 1:4 und besonders bevorzugt 1:3 bis 1:4.

Im erfindungsgemäßen Verfahren beträgt das molare Verhältnis von Ethanol zu inertem organischen Lösungsmittel beispielsweise 1:0,7 bis 1:2,0, bevorzugt 1:0,8 bis 1:1,8 und besonders bevorzugt 1:1,0 bis 1:1,5.

In einer bevorzugten Ausführungsform beträgt somit das Verhältnis von Ethanol zu Toluol 1:0,7 bis 1:2,0, bevorzugt 1:0,8 bis 1:1,8 und besonders bevorzugt 1:1,0 bis 1:1,5.

Es wurde gefunden, dass polare Zusammensetzungen, das heißt Zusammensetzungen mit einem höheren als dem beanspruchten Anteil an Ethanol, die Entstehung von feinen Magnesiumethoxid-Suspensionen begünstigen. Wenn das Verhältnis von Ethanol zu inertem organischen Lösungsmittel 1:≤0,6 ist, das heißt Ethanol im großen Überschuss vorliegt, dann ist die Partikelgröße des hergestellten Magnesiumethoxids klein. Nachteilig daran ist, dass durch die geringe Partikelgröße die Filterporen beim Filtrieren des hergestellten Magnesiumethoxids verstopfen und die Handhabung durch einen hpohen Staubanteil insgesamt erschwert wird.

Zusätzlich erfüllt das hergestellte Magnesiumethoxid nicht die eingangs erwähnte Größenspezifikation für eine optimale Verwendung zur Herstellung von MgCl₂ als Träger für Ziegler-Natta-Katalysatoren.

Unpolare Zusammensetzungen, das heißt Zusammensetzungen mit einem höherem als dem beanspruchten Anteil an inertem organischen Lösungsmittel wie Toluol, begünstigen die Aggregation der hergestellten Magnesiumethoxidpartikel. Wenn das molare Verhältnis von Ethanol zu inertem organischen Lösungsmittel 1:≥2 ist, das heißt das inerte organisch Lösungsmittel in einem großen Überschuss vorliegt, dann kommt es zu einer starken Aggregation, was ebenfalls zur Unbrauchbarkeit des herstellten Magnesiumethanolats führt

Die Zugabe des Ethanols zur erhaltenen Zusammensetzung aus Schritt (1) erfolgt im Rahmen dieser Erfindung beispielsweise über einen Zeitraum von einer Minute bis 10 Stunden, bevorzugt 30 Minuten bis 1,5 Stunden.

Schritt (2) wird bei einer Reaktionstemperatur von 40 bis 70 °C durchgeführt.

Wenn die Reaktionstemperatur zu niedrig ist, dann läuft die Reaktion nicht oder nur sehr langsam ab. Wenn die Reaktionstemperatur zu hoch ist, dann ist die Aggregation des hergestellten Magnesiumethoxids und des eingesetzten Magnesiums aufgrund der zu schnellen Reaktion und der damit einhergehenden zu schnellen H₂-Gasentwicklung begünstigt.

Bevorzugt erfolgt die Zugabe des Ethanols bei einer Temperatur von 50 bis 65 °C.

Schritt (2) kann beispielsweise bei einem Druck von 100 hPa bis 5 MPa durchgeführt werden, bevorzugt bei 900 hPa bis 1500 hPa und besonders bevorzugt bei Umgebungsdruck. Die Reaktionsdauer verringert sich, wenn die Reaktion bei einem Druck über Normaldruck, das heißt über 101 325 Pa durchgeführt wird.

In einer bevorzugten Ausführungsform erfolgt nach Schritt (2) ein Schritt (3) Nachreaktion bei einer Temperatur von 80 bis 110 °C.

Bevorzugt wird nach Beendigung des Schritts (2), das heißt nach der vollständigen Zugabe des Ethanols, im Schritt (3) die Reaktionsmischung bis zu 24 Stunden, besonders bevorzugt für 4 bis 8 Stunden und ganz besonders bevorzugt für 5 bis 6 Stunden bei einer Temperatur von 70 bis 110 °C, bevorzugt bei einer Temperatur von 78bis 83 °C unter Rühren weiter gehalten.

In einer weiteren bevorzugten Ausführungsform erfolgt nach dem Schritt (2) oder vorzugsweise dem Schritt (3) in einem nachfolgenden Schritt (4) die Abtrennung des hergestellten Magnesiumethoxids, bevorzugt durch Filtration oder durch Einengung, besonders bevorzugt durch Filtration.

In einer ebenfalls bevorzugten Ausführungsform erfolgt das nach Schritt (4) in einem nachfolgenden Schritt (5) das ein- oder mehrmalige Waschen des hergestellten Magnesiumethoxids mit inertem organischen Lösungsmittel, bevorzugt mit Hexan.

In einer weiteren bevorzugten Ausführungsform erfolgt in einem nachfolgenden Schritt (6) die Trocknung des hergestellten Magnesiumethoxids, bevorzugt durch Anlegen eines Vakuums. Besonders bevorzugt erfolgt die Trocknung im Vakuum für mindestens 4 Stunden bei 80 bis 110 °C und einem Druck von 100 hPa oder weniger, beispielsweise 1 bis 100 hPa.

Durch Gaseinschlüsse des entstehenden H₂ lagern sich hergestellte MagnesiumethoxidPartikel an der Lösungsmittel-Oberfläche an und werden nicht vollständig mit inertem organischen Lösungsmittel benetzt. Bevorzugt finden daher die Zugabe des Ethanols in Schritt (2) und/oder die Nachreaktion in Schritt (3) unter Rühren statt.

Das Rühren kann beispielsweise durch einen herkömmlichen Rührer, beispielsweise einen Ankerrührer oder einen Propellerrührer, bevorzugt durch einen Propellerrührer erfolgen.

Der Propellerrührer zerreibt, im Gegensatz zum Ankerrührer, auch bei höheren Rührgeschwindigkeiten das hergestellte Magnesiumethoxid nicht, sondern erzeugt eine vertikal zirkulierende Strömung, welche die aufschwimmende Schicht unterrührt. Somit werden Totzonen vermieden und eine möglichst turbulente Durchströmung des Reaktionsgefäßes erzielt.

Bei Verwendung eines Ankerrührers wird bevorzugt ein solcher gewählt, bei dem die Flügel höher sind als der Lösungsmittelstand.

Sowohl die erfindungsgemäße Umsetzung als auch die Gewinnung des erfindungsgemäßen Magnesiumethoxid führt man typischerweise unter Ausschluss von Luft und Feuchtigkeit, bevorzugt unter Schutzgas durch. Ganz besonders bevorzugt setzt man ein getrocknetes sowie CO₂-freies Schutzgas ein, beispielsweise Stickstoff, Argon oder Helium.

Das erfindungsgemäß hergestellte Magnesiumethoxid ist insbesondere zur Herstellung von Ziegler-Natta-Katalysatoren geeignet und bislang als solches noch nicht beschrieben.

Gegenstand der vorliegenden Erfindung ist daher weiterhin Magnesiumethoxid, hergestellt nach dem erfindungsgemäßen Verfahren.

Gegenstand der vorliegenden Erfindung ist weiterhin Magnesiumethoxid mit einem Grobpartikelanteil, ermittelt durch Siebkornfraktionen ≤ 500µm (Maschenweite) von < 40 Gew.-% und > 500 µm mit einem Anteil von > 60 Gew.-%.

Gegenstand der vorliegenden Erfindung ist daher auch ein partikelförmiges Magnesiumethoxid mit einer Reinheit von > 99,0 Gew.-%, einem Partikeldurchmesser von 500 bis 800 µm, freiem EtOH von ≤ 1,0 Gew.-%, bevorzugt von ≤ 0,5 , freiem Magnesium von ≤ 0,5 Gew.-% und Mg(OH)₂ und MgCO₃ von ≤ 1,0 Gew.-%.

Die Erfindung betrifft daher weiterhin die Verwendung des nach den erfindungsgemäßen Verfahren hergestellten Magnesiumethoxids zur Herstellung von Ziegler-Natta-Katalysatoren.

Der Vorteil der Erfindung liegt insbesondere darin, dass das hergestellte Magnesiumethoxid freifließend ist und somit eine einfach Handhabung bei der weiteren Verwendung zur Herstellung von Ziegler-Natta-Katalysatoren möglich ist.

### Beispiele

### Eingesetzte Materialien:

- Magnesiumpulver: Mg (CAS#: 7439-95-4); Partikelgröße: Siebanalyse nach ISO 4497 von 20 bis 5.000 µm
- Magnesiumspäne: Mg (CAS#: 7439-95-4); durchschnittliche Dicke ca. 0,3 mm, durchschnittliche Breite: ca. 1 mm, durchschnittliche Länge: ca. 2-4 mm
- Triethylaluminium: TEA (CAS#: 97-93-8); erhältlich bei LANXESS Organometallics
- Toluol: C₇H₈; (CAS#: 108-88-3); erhältlich bei Sigma-Aldrich
- Ethanol (EtOH): C₂H₅OH; (CAS#: 64-17-5); erhältlich bei Sigma-Aldrich
- Hexan: C₆H₁₄; (CAS#: 110-54-3); erhältlich bei Sigma-Aldrich

### Messmethoden:

### Bestimmung des Magnesium- und Aluminiumgehaltes

Zur Bestimmung des Massenanteils der Metalle wurde eine Probe mit 0,1 molarer wässriger Salzsäure hydrolysiert und vollständig aufgelöst. Die Konzentrationen der Mg- und Al-Ionen in der Lösung wurden über die Messung mittels optischer Emissionsspektroskopie mit einem induktiv gekoppelten Plasma (ICP-OES) bestimmt.

Über die gemessene Konzentration konnte mit dem bekannten Probenvolumen die Stoffmenge und damit die Masse der Metallkationen in der Lösung bestimmt werden. Normiert auf die eingesetzte Probeneinwaage ergab sich der Massenanteil. ICP-OES Messungen wurden mit einem Spectro Arcos FHX22 durchgeführt.

### Messung des Reaktionsumsatzes

Zur genauen Bestimmung des Reaktionsumsatzes wurde eine Probe von Magnesiumethoxid mit HCl hydrolysiert und das Volumen der entstandenen Gasmenge H₂, die bei der Reaktion der Säure mit nicht umgesetztem elementaren Magnesium entsteht, mit einer Gasbürette bestimmt. Die entstandene Stoffmenge an H₂ ist gleich der Stoffmenge des freien Magnesiums.

### Bestimmung des Lösungsmittelgehalts in Magnesiumethoxid

Eine Probe des hergestellten Magnesiumethoxids wurde per Headspace-Probenahme mit anschließender Messung der Gasprobe im Gaschromatographen (GC) vermessen. Der Feststoff wurde in einer Glasampulle für 30 Minuten auf 80 °C erhitzt und eine Probe der Gasphase entnommen und per GC gemessen. Die gaschromatographischen Analysen wurden mit einem Chromatographen 7890A (Agilent Technologies Inc.) aufgenommen.

Soweit nicht anders vermerkt, wurden alle Reaktionen unter Argon-Atmosphäre mit Schlenktechnik durchgeführt.

### Beispiel 1 - Synthese von Mg(OEt)₂: Keine Aktivierung (nicht erfindungsgemäß)

Magnesiumspäne (10,00 g; 0,412 mol; 1,00 eq.) wurden in einem 500 mL Dreihalskolben mit ca. 10 mL Ethanol in Toluolsuspension vorgelegt und auf 70 °C geheizt. Nach 30 Minuten wurde die flüssige Phase trüb und es setzte eine leichte, schneller werdende Gasentwicklung ein. Der Rest Ethanol (insgesamt 72,08 mL; 1,238 mol; 3,00 eq.) wurde in 30 Minuten zugetropft und für 4,5 Stunden unter Rückfluss erhitzt. Das hergestellte Magnesiumethoxid wurde zweimal mit Hexan gewaschen und in Hexan über Nacht stehen gelassen, wobei sich eine obere graue und eine untere etwas helle weiße Magnesiumethoxidschicht ausbildete. Nach Trocknung für 5 Stunden im Vakuum wurde für beide Phasen der Mg-Wert bestimmt.

### Beispiel 2 - Synthese von Mg(OEt)₂: Magnesiumpulver und Ethanol

Magnesiumpulver (10,00 g; 0,412 mol; 1,00 eq.) wurden in einem 500 mL Dreihalskolben in Toluol (214,00 mL; 1,970 mol; 4,78 eq.) suspendiert, mit TEA (15,00 mL; 0,028 mol; 0,07 eq.; 25 Gew.-% in Toluol) versetzt und eine Stunde bei 50°C gerührt. Außerdem wurden 102 g Glaskugeln mit einem Durchmesser von 4 mm zugegeben. Ethanol (50,40 mL; 0,864 mol; 2,10 eq.) wurde langsam bei 55-65 °C in 2,5 Stunden zugetropft. Es wurde 5 Stunden bei Rückfluss gerührt. Nach kurzer Zeit bei Rückfluss war das hergestellte Magnesiumethoxid vollständig aggregiert. Es wurde grob zerkleinert, mit dreimal 50 mL Hexan gewaschen und im Vakuum für 2 Stunden getrocknet. Das hergestellte Magnesiumethoxid lag als grauer Feststoff mit sichtbaren metallischen Rückständen vor.

### Beispiel 3 - Synthese von Mg(OEt)₂: Magnesiumspäne und Ethanol (nicht erfindungsgemäß; siehe auch DE-A-102006041315)

Magnesiumspäne (10,00 g; 0,412 mol; 1,00 eq.) wurden in einem 500 mL Dreihalskolben in Toluol (214,00 mL; 1,970 mol; 4,78 eq.) suspendiert, mit TEA (15,00 mL; 0,028 mol; 0,07 eq.; 25 Gew.--% in Toluol) versetzt und eine Stunde bei 50°C gerührt. Ethanol (50,40 mL; 0,864 mol; 2,10 eq.) wurde langsam bei 55-65 °C in 45 Minuten zugetropft. Es wurde 2,5 Stunden bei Rückfluss gerührt. Nach kurzer Zeit bei Rückfluss war das hergestellte Magnesiumethoxid vollständig aggregiert. Es wurde grob zerkleinert, filtriert, dreimal mit 50 mL Hexan gewaschen und im Vakuum für 2 Stunden getrocknet. Das Produkt lag als grauer Feststoff mit sichtbaren metallischen Rückständen vor.

### Beispiele 4-8 - Synthese von Mg(OEt)₂: Optimierung der Lösungsmittelmengen

Magnesiumspäne (10,00 g; 0,412 mol; 1,00 eq.) wurden in einem 500 mL Dreihalskolben mit 2-5 mol-% TEA versetzt und eine Stunde bei 50 °C gerührt. Ethanol wurde in 45 Minuten bei 50-60 °C zugetropft. Danach wurde zur Nachreaktion auf 80 °C erhitzt und das hergestellte Magnesiumethoxid filtriert, dreimal mit 50 mL Hexan gewaschen und für 2-3 Stunden im Vakuum getrocknet. Die variierten Bedingungen und Analysenergebnisse sind in den Tabellen 1 und 2 zusammengefasst.

Bei einer sehr hohen EtOH-Menge (Beispiel 4) wird die flüssige Phase im Verlauf der Zugabe zu unpolar wodurch eine Phasentrennung und damit einhergehende Aggregation begünstigt wird. Der Umsatz ist unvollständig.

Bei einer sehr geringen EtOH-Menge (Beispiel 2 und 3) wird die Reaktionsgeschwindigkeit sehr klein. In Beispiel 5 bilden sich zunächst noch runde Partikel, die zu einer milchig weißen Suspension zerfallen. Das hergestellte Magnesiumethoxid ist sehr fein und weist einen hohen Staubanteil auf.

Wenn die Toluol-Menge im Vergleich zu Ethanol zu gering ist, das heißt <1 Äquivalent, dann liegt das hergestellte Magnesiumethoxid als feines Pulver vor mit dem Nachteil, dass dies beim Abfiltrieren die Filterporen verstopft.

Wenn die Toluol-Menge im Vergleich zu Ethanol zu groß ist, das heißt >2,3 Äquivalente, dann kommt es zur Aggregation des hergestellte Magnesiumethoxids.

### Beispiele 8-10 - Synthese von Mg(OEt)₂: Optimierung der Aktivator-Mengen

Magnesiumspäne wurden (10,00 g; 0,412 mol; 1,00 eq.) wurden in einem 500 mL Dreihalskolben in Toluol (150,00 mL; 1,416 mol; 3,44 eq.) vorgelegt und mit unterschiedlichen Mengen TEA versetzt und eine Stunde bei 50 °C gerührt. Ethanol (72,08 mL; 1,238 mol; 3,00 eq.) wurde in 45 Minuten bei 50-60 °C zugetropft. Danach wurde zur Nachreaktion auf 80 °C erhitzt und das hergestellte Magnesiumethoxid dreimal mit 50 mL Hexan gewaschen und für 2-3 Stunden im Vakuum getrocknet. Die variierten Bedingungen, Mengen an TEA und Analysenergebnisse sind in den Tabellen 1 bis 3 zusammengefasst.

**Tabelle 1: Eingesetzte Komponenten der Beispiele 1 bis 10**

| **Bsp.** | **Mg [g]** | **TEA [mL]** | **EtOH [mL]** | **Toluol [mL]** | **Mg [eq.]** | **TEA [eq.]** | **EtOH [eq.]** | **Toluol [eq.]** | **n(EtOH): n(Toluol)** |
|---|---|---|---|---|---|---|---|---|---|
| **1*** | 10 | - | 72,08 | 150 | 1 | - | 3 | 3.44 | 1:1.1 |
| **2*** | 10 | 15 | 50,4 | 214 | 1 | 0,07 | 2,1 | 4,78 | 1:2,3 |
| **3*** | 10 | 15 | 50,4 | 214 | 1 | 0,07 | 2,1 | 4,78 | 1:2,3 |
| **4*** | 10 | 10 | 151,2 | 50 | 1 | 0,05 | 6,3 | 1,15 | 1:0,2 |
| **5*** | 10 | 10 | 96,11 | 100 | 1 | 0,05 | 4 | 2,29 | 1:0,6 |
| **6** | **10** | **5** | **72,08** | **150** | 1 | **0,02** | 3 | 3,44 | 1:1,1 |
| **7*** | 10 | 5 | 72,08 | 300 | 1 | 0,02 | 3 | 6,88 | 1:2,3 |
| **8** | **10** | **5** | **72,08** | **150** | 1 | **0,02** | 3 | 3,44 | 1:1,1 |
| **9** | **10** | **1,09** | **72,08** | **150** | 1 | **0,005** | 3 | 3,44 | 1:1,1 |
| 10 | **10** | **0,22** | **72,08** | 150 | **1** | **0,001** | **3** | **3,44** | **1:1,1** |

| | | | | | | | | | |
|---|---|---|---|---|---|---|---|---|---|
| * Beispiele 1-5 und 7: Nicht erfindungsgemäß, zum Vergleich Beispiele 6 und 8-10: Erfindungsgemäß | | | | | | | | | |

**Tabelle 2: Reaktionsbedingungen der Beispiele 1 bis 10**

| **Bsp.** | **Reaktionszeit [min]** | **Reakions-Temp. [°C]** | **Nachreaktion [h]** | **Trocknung [h]** | **Vakuum** |
|---|---|---|---|---|---|
| **1** | 30 | 70 | 4,5 | 5 | Ja |
| **2** | 150 | 50 | 5 | 2 | Ja |
| **3** | 45 | 50 | 2,5 | 2 | Ja |
| **4** | 45 | 55-65 | 10 (80°C) | 2-3 | Ja |
| **5** | 45 | 55-60 | 6 (80°C) | 2-3 | Ja |
| **6** | **45** | **55-60** | **6 (80°C)** | **2-3** | **Ja** |
| **7** | 45 | 55-60 | 4 (85°C) | 2-3 | Ja |
| **8** | **45** | **50-60** | **6 (78-83°C)** | **2-3** | **Ja** |
| **9** | **45** | **50-60** | **8 (78-83°C)** | **3,5** | **Ja** |
| **10** | **45** | **50-60** | **4,8 (78-83°C)** | **6** | **Ja** |

**Tabelle 3: Analysenergebnisse der Beispiele 1 bis 10**

| **Bsp.** | **Aggregation Morphologie** | **Mg-Wert [Gew.-%]** | **AI-Wert [Gew.-%]** |
|---|---|---|---|
| **1** | vollständig | 20,62 * | - |
| | | 20,56 ** | |
| **2** | vollständig | 20,21 | 1,04 |
| **3** | vollständig | 21,08 | 1,27 |
| **4** | feines weißes Pulver | 19,4 | 0,16 |
| **5** | feines weißes Pulver | 19,62 | 0,2 |
| **6** | **nicht aggregierte, weiße Kügelchen/ Plättchen,** | **18,67** | **0,39** |
| **7** | etwas Aggregation weiß-graue Kügelchen, | 19,94 | 0,46 |
| **8** | **nicht aggregierte, weiße bis weiß-graue Partikel** | **18,67** | **0,391** |
| **9** | **nicht aggregierte, weiße bis weiß-graue Partikel** | **20,65** | **0,122** |
| **10** | **nicht aggregierte,weiße bis weiß-graue Partikel** | **21,27** | **0,03** |

| | | | |
|---|---|---|---|
| * Obere graue Phase; ** Untere weiße Phase | | | |

## Patentansprüche

1. Verfahren zur Herstellung von Magnesiumethoxid umfassend die Schritte
(1) Inkontaktbringen von elementarem Magnesium mit zumindest einem Aluminiumalkyl in Gegenwart von inertem organischen Lösungsmittel und
(2) Zugabe von Ethanol bei einer Temperatur von 40 bis 70 °C, bevorzugt 50 bis 65 °C,
wobei das molare Verhältnis von Ethanol zu inertem organischen Lösungsmittel von 1:0,7 bis 1:2, bevorzugt von 1:0,8 bis 1:1,8 und besonders bevorzugt von 1:1 bis 1:1,5 beträgt.

2. Verfahren nach Anspruch 1, **dadurch gekennzeichnet, dass** das in Schritt (1) eingesetzte elementare Magnesium in Form von Magnesiumpulver, Magnesiumgranulat oder Magnesiumspänen, bevorzugt in Form von Magnesiumspänen eingesetzt wird.

3. Verfahren nach Anspruch 1 oder 2, **dadurch gekennzeichnet, dass** das in Schritt (1) eingesetzte inerte organische Lösungsmittel ausgewählt ist aus der Gruppe aromatische oder aliphatische Kohlenwasserstoffe oder Mischungen davon, wobei Toluol, n-Octan, Heptane; Hexane, Cyclohexan, Pentane, Paraffinöle oder beliebige Mischungen davon bevorzugt sind, und Toluol besonders bevorzugt ist.

4. Verfahren nach einem der Ansprüche 1 bis 3, **dadurch gekennzeichnet, dass** die Menge an inertem organischen Lösungsmittel beispielsweise 1 bis 10 Mol-Äquivalente, bevorzugt 2 bis 7 Mol-Äquivalente und besonders bevorzugt 2 bis 4 Mol-Äquivalente bezogen auf die Menge an eingesetztem Magnesium beträgt.

5. Verfahren nach einem der Ansprüche 1 bis 4, **dadurch gekennzeichnet, dass** die eingesetzten Aluminiumalkyle ausgewählt sind aus der Gruppe bestehend aus Trimethylaluminium, Triethylaluminium, Tributylaluminium, Triisobutylaluminium, Ethylaluminiumdichlorid und Diethylaluminiumchlorid oder Mischungen der vorgenannten Aluminiumalkyle, wobei Triethylaluminium bevorzugt ist.

6. Verfahren nach einem der Ansprüche 1 bis 5, **dadurch gekennzeichnet, dass** die Menge an eingesetztem Aluminiumalkyl beispielsweise 0,001 bis 0,1 Mol-Äquivalente, bevorzugt 0,6 bis 0,8 Mol-Äquivalente bezogen auf die Menge Magnesium beträgt.

7. Verfahren nach einem der Ansprüche 1 bis 6, **dadurch gekennzeichnet, dass** das in schritt (2) eingesetzte Ethanol einen Wassergehalt von weniger als 0,05 Gew.- %, besonders bevorzugt weniger als 0,01 Gew.-% und ganz besonders bevorzugt weniger als 0,01 Gew.-% bezogen auf das Gesamtgewicht des Ethanols aufweist.

8. Verfahren nach einem der Ansprüche 1 bis 7, **dadurch gekennzeichnet, dass** das Verhältnis von Magnesium zu Ethanol 1:2,1 bis 1:4, bevorzugt 1:2,5 bis 1:4 und besonders bevorzugt 1:3 bis 1:4 beträgt.

9. Verfahren nach einem der Ansprüche 1 bis 8, **dadurch gekennzeichnet, dass** die Zugabe des Ethanols in Schritt (2) über einen Zeitraum von einer Minute bis 10 Stunden, bevorzugt 30 Minuten bis 1,5 Stunden erfolgt.

10. Verfahren nach einem der Ansprüche 1 bis 9, **dadurch gekennzeichnet, dass** nach Schritt (2) noch als Schritt (3) die Nachreaktion bei einer Temperatur von 80 bis 110 °C, bevorzugt von 70 bis 110 °C, besonders bevorzugt bei einer Temperatur von 78 bis 83 °C erfolgt.

11. Partikelförmiges Magnesiumethoxid mit einer Reinheit von ≥ 99,0 Gew.-%, einem Partikeldurchmesser von 500 bis 800 µm, einem Gehalt an freiem EtOH von ≤ 1,0 Gew.-%, bevorzugt von ≤ 0,5 , freiem Magnesium von ≤ 0,5 Gew.-% und Mg(OH)₂ und MgCO₃ von ≤ 1,0 Gew.-%.

12. Verwendung von Magnesiumethoxid nach Anspruch 11 oder das nach einem Verfahren gemäß einem der Ansprüche 1 bis 10 hergestellt wurde zur Herstellung von Ziegler-Natta-Katalysatoren.

13. Verfahren zur Herstellung von Ziegler-Natta-Katalysatoren **dadurch gekennzeichnet, dass** Magnesiumethoxid nach Anspruch 11 oder das nach einem Verfahren gemäß einem der Ansprüche 1 bis 10 hergestellt wurde, eingesetzt wird.
